# EUROPÄISCHE PATENTSCHRIFT

(11) **EP 1 610 893 B1**
(45) Veröffentlichungstag und Bekanntmachung des Hinweises auf die Patenterteilung: **07.03.2007**
(21) Anmeldenummer: 04722843.2
(22) Anmeldetag: 24.03.2004
(51) Int. Cl.: B01J 27/14

(54) **ALS KATALYSATOR FÜR DIE HYDROCYANIERUNG VON OLEFINISCH UNGESÄTTIGTEN VERBINDUNGEN GEEIGNETES SYSTEM**
SYSTEM SUITABLE FOR THE HYDROCYANATION OF OLEFINICALLY UNSATURATED COMPOUNDS
SYSTEME APPROPRIE COMME CATALYSEUR POUR L'HYDROCYANATION DE COMPOSES OLEFINIQUEMENT INSATURES

(30) Priorität: 31.03.2003 DE 10314761
(43) Veröffentlichungstag der Anmeldung: 04.01.2006
(73) Patentinhaber: BASF Aktiengesellschaft, 67056 Ludwigshafen (DE)
(72) Erfinder: BARTSCH, Michael, 67433 Neustadt (DE); BAUMANN, Robert, 68159 Mannheim (DE); HADERLEIN, Gerd, 67269 Grünstadt (DE); FLORES, Miguel Angel, E-28300 Aranjuez (ES); JUNGKAMP, Tim, B-2950 Kapelle (BE); LUYKEN, Hermann, 67069 Ludwigshafen (DE); SCHEIDEL, Jens, 69493 Hirschberg (DE); SIEGEL, Wolfgang, 67117 Limburgerhof (DE); MOLNAR, Ferenc, 67346 Speyer (DE)
(86) Internationale Anmeldenummer: PCT/EP2004/003103
(87) Internationale Veröffentlichungsnummer: WO 2004/087314

(56) Entgegenhaltungen:
- EP-A- 0 268 448
- US-A- 3 565 967
- US-A- 4 025 570
- US-A- 5 169 971

## Beschreibung

Die vorliegende Erfindung betrifft ein als Katalysator für die Hydrocyanierung von olefinisch ungesättigten Verbindungen geeignetes System, enthaltend
a) Ni(0)
b) Ni(0) als Ligand komplexierende, dreibindigen Phosphor enthaltende Verbindung,
c) eine Lewis-Säure
   und
d) eine Verbindung der Formel M Rₙ
wobei
- M:: Al oder Ti
- R:: gleiche oder unterschiedliche einwertige Alkoxyreste, wobei mehrere Alkoxyreste untereinander verbrückt sein können, zusätzlich im Falle von M = Al R gleiche oder unterschiedliche einwertige Alkylreste, wobei mehrere Alkylreste untereinander verbrückt sein können oder ein oder mehrere Alkylreste mit einem oder mehreren der oben genannten Alkoxyresten verbrückt sein können
- n:: Wertigkeit von M
darstellen.

Weiterhin betrifft sie ein Verfahren zur Hydrocyanierung einer olefinisch ungesättigten Verbindung in Gegenwart eines solchen Systems.

Verfahren zur Hydrocyanierung eines olefinisch ungesättigten Nitrils, insbesondere die Herstellung von Adipodinitril durch Hydrocyanierung einer olefinisch ungesättigten Verbindung wie 2-cis-Pentennitril, 2-trans-Pentennitril, 3-cis-Pentennitril, 3-trans-Pentennitril, 4-Pentennitril, E-2-Methyl-2-butennitril, Z-2-Methyl-2-butennitril, 2-Methyl-3-butennitril oder deren Gemische, in Gegenwart eines Katalysatorsystems, enthaltend eine Lewis-Säure und eine Komplexverbindung enthaltend eine als Ligand geeignete phosphorhaltige Verbindung wie eine monodentate, vorzugsweise multidentate, insbesondere bidentate Verbindung, deren Koordination mit einem Zentralatom über ein Phosphoratom erfolgt, das als Phosphin, Phosphit, Phosphonit oder Phosphinit oder deren Gemische vorliegen kann, und ein Zentralatom, vorzugsweise Nickel, Kobalt oder Palladium, insbesondere Nickel, besonders bevorzugt in Form von Nickel-(0), sind bekannt, beispielsweise aus US 3,496,217, US 3,496,218, US 4,705,881, US 4,774,353, US 4,874,884, US 5,773,637, US 6,127,567, US 6,171,996 B1 und US 6,380,421 B1.

Der vorliegenden Erfindung lag die Aufgabe zugrunde, ein als Katalysator für die Hydrocyanierung von olefinisch ungesättigten Verbindungen geeignetes System bereitzustellen, das eine gegenüber den bekannten Systemen verbesserte Raum-Zeit-Ausbeute an Produkt der Hydrocyanierung aufweist.

Demgemäß wurde das eingangs definierte System sowie ein Verfahren zur Hydrocyanierung einer olefinisch ungesättigten Verbindung in Gegenwart eines solchen Systems gefunden.

Die Herstellung von Ni(0) enthaltenden Katalysatorsystemen ist an sich bekannt und kann für die Zwecke der vorliegenden Erfindung nach an sich bekannten Verfahren erfolgen.

Weiterhin enthält das System zusätzlich eine als Ligand für Ni(0) geeignete Verbindung, die mindestens ein dreibindiges Phosphoratom aufweist, oder ein Gemisch solcher Verbindungen.

In einer bevorzugten Ausführungsform kann als solche als Ligand geeignete Verbindung eine solche der Formel

P (X¹R¹) (X²R²) (X³R³) (I)

eingesetzt werden.

Unter dieser Verbindung wird im Sinne der vorliegenden Erfindung eine einzelne Verbindung oder ein Gemisch verschiedener Verbindungen der vorgenannten Formel verstanden.
- x¹, X², X³: können unabhängig voneinander Sauerstoff oder Einzelbindung darstellen.

Falls alle der Gruppen X¹, X² und X³ für Einzelbindungen stehen, so stellt Verbindung (I) ein Phosphin der Formel P(R¹ R² R³) mit den für R¹, R² und R³ in dieser Beschreibung genannten Bedeutungen dar.

Falls zwei der Gruppen X¹, X² und X³ für Einzelbindungen stehen und eine für Sauerstoff, so stellt Verbindung (I) ein Phosphinit der Formel P(OR¹)(R²)(R³) oder P(R¹)(OR²)(R³) oder P(R¹)(R²)(OR³) mit den für R¹, R² und R³ in dieser Beschreibung genannten Bedeutungen dar.

Falls eine der Gruppen X¹, X² und X³ für eine Einzelbindung steht und zwei für Sauerstoff, so stellt Verbindung (I) ein Phosphonit der Formel P(OR¹)(OR²)(R³) oder P(R¹)(OR²)(OR³) oder P(OR¹)(R²)(OR³) mit den für R¹, R² und R³ in dieser Beschreibung genannten Bedeutungen dar.

In einer bevorzugten Ausführungsform sollten alle der Gruppen X¹, X² und X³ für Sauerstoff stehen, so daß Verbindung (1) vorteilhaft ein Phosphit der Formel P(OR¹)(OR²)(OR³) mit den für R¹, R² und R³ in dieser Beschreibung genannten Bedeutungen darstellt.

Erfindungsgemäß stehen R¹, R², R³ unabhängig voneinander für gleiche oder unterschiedliche organische Reste.

Als R¹, R² und R³ kommen unabhängig voneinander Alkylreste, vorteilhaft mit 1 bis 10 C-Atomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorteilhaft mit 1 bis 20 C-Atomen, wie 1,1'-Biphenol, 1,1'-Binaphthol in Betracht.

Die Gruppen R¹, R² und R³ können miteinander direkt, also nicht allein über das zentrale Phosphor-Atom, verbunden sein. Vorzugsweise sind die Gruppen R¹, R² und R³ nicht miteinander direkt verbunden.

In einer bevorzugten Ausführungsform kommen als Gruppen R¹, R² und R³ Reste ausgewählt aus der Gruppe bestehend aus Phenyl, o-Tolyl, m-Tolyl und p-Tolyl in Betracht.

In einer besonders bevorzugten Ausführungsform sollten dabei maximal zwei der Gruppen R¹, R² und R³ Phenyl-Gruppen sein.

In einer anderen bevorzugten Ausführungsform sollten dabei maximal zwei der Gruppen R¹, R² und R³ o-Tolyl-Gruppen sein.

Als besonders bevorzugte Verbindungen können solche der Formel

(o-Tolyl-O-)_{w} (m-Tolyl-O-)ₓ (p-Tolyl-O-)_{y} (Phenyl-O-)_{z} P

mit w, x, y, z eine natürliche Zahl
mit w + x + y + z = 3 und
w, z kleiner gleich 2
eingesetzt werden, wie (p-Tolyl-O-)(Phenyl)₂P, (m-Tolyl-O-)(Phenyl)₂P, (o-Tolyl-O-) (Phenyl)₂P, (p-Tolyl-O-)₂(Phenyl)P, (m-Tolyl-O-)₂(Phenyl)P, (o-Tolyl-O-)₂(Phenyl)P, (m-Tolyl-O-)(p-Tolyl-O-)(Phenyl)P, (o-Tolyl-O-)(p-Tolyl-O-)(Phenyl)P, (o-Tolyl-O-) (m-Tolyl-O-)(Phenyl)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Tolyl-O-)P, (o-Tolyl-O-)₂(p-Tolyl-O-)P, (o-Tolyl-O-)(m-Tolyl-O-) (p-Tolyl-O-)P, (m-Tolyl-O-)₃P, (o-Tolyl-O-)(m-Tolyl-O-)₂P (o-Tolyl-O-)₂(m-Tolyl-O-)P oder Gemische solcher Verbindungen eingesetzt werden.

So können beispielsweise Gemische enthaltend (m-Tolyl-O-)₃P, (m-Tolyl-O-)₂(p-Tolyl-O-)P, (m-Tolyl-O-)(p-Tolyl-O-)₂P und (p-Tolyl-O-)₃P durch Umsetzung eines Gemisches enthaltend m-Kresol und p-Kresol, insbesondere im Molverhältnis 2:1, wie es bei der destillativen Aufarbeitung von Erdöl anfällt, mit einem Phosphortrihalogenid, wie Phosphortrichlorid, erhalten werden.

Solche Verbindungen und deren Herstellung sind an sich bekannt.

In einer weiteren bevorzugten Ausführungsform können als für Ni(0) als Ligand geeignete Verbindung solche der Formel 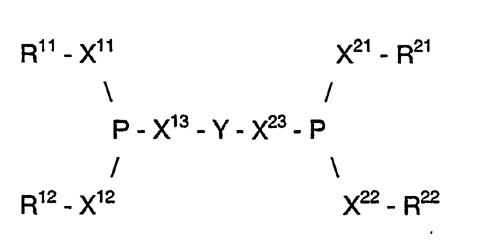 mit
- X¹¹, X¹², X¹³ X²¹, X²², X²³: unabhängig voneinander Sauerstoff oder Einzelbindung
- R¹¹, R¹²: unabhängig voneinander gleiche oder unterschiedliche, einzelne oder verbrückte organische Reste
- R²¹, R²²: unabhängig voneinander gleiche oder unterschiedliche, einzelne oder verbrückte organische Reste,
- Y: Brückengruppe
eingesetzt werden.

Unter einer solchen Verbindung wird im Sinne der vorliegenden Erfindung eine einzelne Verbindung oder ein Gemisch verschiedener Verbindungen der vorgenannten Formel verstanden.

In einer bevorzugten Ausführungsform können X¹¹, X¹², X¹³ X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so daß das mit X¹¹, X¹² und X¹³ umgebene Phosphoratom Zentralatom eines Phosphonits ist. In einem solchen Fall können X²¹ , X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so daß das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzelbindung oder X¹¹ Sauerstoff und X¹² und X¹³ eine Einzelbindung darstellen, so daß das mit X¹¹, X¹² und X¹³ umgebene Phosphoratom Zentralatom eines Phosphinits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so daß das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so daß das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so daß das mit X²¹, X²² und X²³ umgebene Phosphoratom Zentralatom eines Phosphits oder Phosphins, vorzugsweise eines Phosphins, sein kann.

Als Brückengruppe Y kommen vorteilhaft substituierte, beispielsweise mit C₁-C₄-Alkyl, Halogen, wie Fluor, Chlor. Brom, halogeniertem Alkyl, wie Trifluormethyl, Aryl, wie Phenyl, oder unsubstituierte, Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, inbesondere durch C₁-C₄-Alkyl, Halogen, wie Fluor, Chlor. Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterschiedliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, inbesondere durch C₁-C₄-Alkyl, Halogen, wie Fluor, Chlor. Brom, halogeniertem Alkyl, wie Trifluormethyl, Aryl, wie Phenyl, oder unsubstituierte Arylgruppen.

Die Reste R¹¹ und R¹² können einzeln oder verbrückt sein.
Die Reste R²¹ und R²² können einzeln oder verbrückt sein.
Die Reste R¹¹, R¹², R²¹ und R²² können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht.

In einer besonders bevorzugten Ausführungsform kommen die in US 5,512,696 genannten Verbindungen der Formel I, II, III, IV, V, V) und VII, insbesondere die dort in den Beispielen 1 bis 31 eingesetzte Verbindungen, in Betracht.

In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzte Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzte Verbindungen, in Betracht.

In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzte Verbindungen, in Betracht.

In einer besonders bevorzugten Ausführungsform kommen die in US 6,127,567 genannten Verbindungen und dort in den Beispielen 1 bis 29 eingesetzte Verbindungen in Betracht.

In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzte Verbindungen, in Betracht:

In einer besonders bevorzugten Ausführungsform kommen die in US 5,959,135 genannten Verbindungen und dort in den Beispielen 1 bis 13 eingesetzte Verbindungen in Betracht.

In einer besonders bevorzugten Ausführungsform kommen die in US 5,847,191 genannten Verbindungen der Formel I, II und III in Betracht.

In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 12, 13, 14, 15, 16, 17, 18, 19, 20 und 21 dargestellten Verbindungen, in Betracht.

In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht.

In einer besonders bevorzugten Ausführungsform kommen die in WO 99/13983 genannten Verbindungen in Betracht.

In einer besonders bevorzugten Ausführungsform kommen die in WO 99/64155 genannten Verbindungen in Betracht.

In einer besonders bevorzugten Ausführungsform kommen die in der deutschen Offenlegungsschrift DE 10038037 genannten Verbindungen in Betracht.

In einer besonders bevorzugten Ausführungsform kommen die in der deutschen Offenlegungsschrift DE 10046025 genannten Verbindungen in Betracht.

Solche Verbindungen und deren Herstellung sind an sich bekannt.

In einer weiteren bevorzugten Ausführungsform kann ein Gemisch einer oder mehrerer der vorgenannten als Ligand für Ni(0) geeigneten Verbindungen, die ein Phosphoratom enthalten, und einer oder mehrer als Ligand für Ni(0) geeigneten Verbindungen, die zwei Phosphoratome enthalten, eingesetzt werden.

Hierbei kann insbesondere das Verhältnis der ersten Komponente zu der zweiten Komponente im Bereich von 4/1 bis 1/1 mol/mol liegen.

In einer besonders bevorzugten Ausführungsform kommen die in der internationalen Patentanmeldung PCT/EP02/07888 genannten Ni(0) und solche Gemische enthaltenden Systeme in Betracht.

Weiterhin enthält das System eine Lewis-Säure durchgeführt werden.

Im Sinne der vorliegenden Erfindung wird unter einer Lewis-Säure eine einzelne Lewis-Säure, wie auch ein Gemisch aus mehreren, wie zwei, drie oder vier Lewis-Säuren, verstanden.

Als Lewis-Säure kommen dabei anorganische oder organische Metall-Verbindungen in Betracht, in denen das Kation ausgewählt ist aus der Gruppe bestehend aus Scandium, Titan, Vanadium, Chrom, Mangan, Eisen, Kobalt, Kupfer, Zink, Bor, Aluminium, Yttrium, Zirkonium, Niob, Molybdän, Cadmium, Rhenium und Zinn. Beispiele umfassen ZnBr₂, Znl₂, ZnCl₂, ZnSO₄, CuCl₂, CuCl, Cu(O₃SCF₃)₂, CoCl₂, Col₂, Fel₂, FeCl₃, FeCl₂, FeCl₂(THF)₂, TiCl₄(THF)₂, TiCl₄, TiCl₃, ClTi(O-i-Propyl)₃, MnCl₂, ScCl₃, AlCl₃, (C₈H₁₇)AlCl₂, (C₈H₁₇)₂AlCl, (i-C₄H₉)₂AlCl, (C₆H₅)₂AlCl, (C₆H₅)AlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, TaCl₅, wie sie beispielsweise in US 6,127,567, US 6,171,996 und US 6,380,421 beschrieben sind. Weiterhin kommen in Betracht Metallsalze, wie ZnCl₂, Col₂ und SnCl₂ und organometallische Verbindungen, wie RAlCl₂, R₂AlCl, RSnO₃SCF₃ und R₃B, wobei R eine Alkyl- oder Aryl-Gruppe ist, wie sie beispielsweise in US 3,496,217, US 3,496,218 und US 4,774,353 beschrieben sind. Weiterhin können gemäß US 3,773,809 als Promotor ein Metall in kationischer Form, ausgewählt aus der Gruppe bestehend aus Zink, Cadmium, Beryllium, Aluminium, Gallium, Indium, Thallium, Titan, Zirkonium, Hafnium, Erbium, Germanium, Zinn, Vanadium, Niob, Scandium, Chrom, Molybdän, Wolfram, Mangan, Rhenium, Palladium, Thorium, Eisen und Kobalt, vorzugsweise Zink, Cadmium, Titan, Zinn, Chrom, Eisen, Aluminium und Kobalt, eingesetzt werden, wobei der anionische Teil der Verbindung ausgewählt sein kann aus der Gruppe bestehend aus Halogeniden, wie Fluorid, Chlorid, Bromid und Jodid, Anionen niedriger Fettsäuren mit von 2 bis 7 Kohlenstoffatomen, HPO₃²⁻, H₃PO²⁻, CF₃COO-, C₇H₁₅OSO₂⁻ oder SO₄²⁻. Weiterhin sind aus US 3,773,809 als geeignete Promotoren Borhydride, Organoborhydride und Borsäureester der Formel R₃B und B(OR)₃, wobei R ausgewählt ist aus der Gruppe bestehend aus Wasserstoff, Aryl-Radikale mit zwischen 6 und 18 Kohlenstoff-Atomen, mit Alkyl-Gruppen mit 1 bis 7 Kohlenstoff-Atomen substituierte Aryl-Radikale und mit Cyano-substituierte Alkyl-Gruppen mit 1 bis 7 Kohlenstoff-Atomen substituierte Aryl-Radikale, vorteilhaft Triphenylbor, genannt. Weiterhin können, wie in US 4,874,884 beschrieben, synergistisch wirksame Kombinationen von Lewis-Säuren eingesetzt werden, um die Aktivität des Katalysatorsystems zu erhöhen. Geeignete Promotoren können beispielsweise aus der Gruppe bestehend aus CdCl₂, FeCl₂, ZnCl₂, B(C₆H₅)₃ und (C₆H₅)₃SnX, mit X=CF₃SO₃, CH₃C₆H₄SO₃ oder (C₆H₅)₃BCN ausgewählt werden, wobei für das Verhältnis von Promotor zu Nickel ein Bereich von vorzugsweise etwa 1:16 bis etwa 50:1 genannt ist.

Im Sinne der vorliegenden Erfindung umfasst der Begriff Lewis-Säure auch die in US 3,496,217, US 3,496,218, US 4,774,353, US 4,874,884, US 6,127,567, US 6,171,996 und US 6,380,421 genannten Promotoren.

Als besonders bevorzugte Lewis-Säuren kommen unter den genannten insbesondere Metallsalze, besonders bevorzugt Metallhalogenide, wie Fluoride, Chloride, Bromide, Jodide, insbesondere Chloride, in Betracht, von denen wiederum Zinkchlorid, Eisen-(II)-Chlorid und Eisen-(III)-chlorid besonders bevorzugt sind.

Erfindungsgemäß enthält das System eine Verbindung d) der Formel M Rₙ
wobei
- M:: Al oder Ti
- R:: gleiche oder unterschiedliche einwertige Alkoxyreste, wobei mehrere Alkoxyreste untereinander verbrückt sein können, zusätzlich im Falle von M = Al R gleiche oder unterschiedliche einwertige Alkylreste, wobei mehrere Alkylreste untereinander verbrückt sein können oder ein oder mehrere Alkylreste mit einem oder mehreren der oben genannten Alkoxyresten verbrückt sein können
- n:: Wertigkeit von M
darstellen.

Im Sinne der vorliegenden Erfindung wird unter einer Verbindung d) eine einzelne Verbindung, wie auch ein Gemisch verschiedener solcher Verbindungen verstanden, wobei sich die verschiedenen Verbindungen in der Art von M, der Art von R oder beidem unterscheiden können.

Erfindungsgemäß steht M für Aluminium oder Titan, wobei die Wertigkeit n von Aluminium in Verbindung d) vorteilhaft drei und die Wertigkeit n von Titan in Verbindung d) vorteilhaft drei oder vier, insbesondere vier betragen sollte. Unter der Wertigkeit wird im Sinne der Definition von n die Zahl der Reste R an M verstanden, unabhängig von der sich für die jeweilige Struktur M Rₙ in Verbindung d) errechenbaren Oxidationszahl von M.

Für den Fall, dass M für Titan steht, steht R für gleiche oder unterschiedliche, vorzugsweise gleiche, einwertige Alkoxyreste, wobei mehrere Alkoxyreste untereinander verbrückt sein können, vorzugsweise für C₁-C₄-Alkoxyreste, wie Methoxy, Ethoxy, 1-Propoxy, 2-Propoxy, 1-n-Butoxy, 2-n-Butoxy, 1-i-Butoxy oder 2-i-Butoxy, vorzugsweise Ti(OMe)₄, Ti(OEt)₄, Ti(O-i-Pr)₄, Ti(O-n-Pr)₄, insbesondere Ti(O-i-Pr)₄

In einer bevorzugten Ausführungsform kann Verbindung d) ein Titantetraalkoxylat, insbesondere Ti(O-i-Pr)₄ darstellen.

Für den Fall, dass M für Aluminium steht, steht R für gleiche oder unterschiedliche, vorzugsweise gleiche, einwertige Alkoxyreste, wobei mehrere Alkoxyreste untereinander verbrückt sein können, vorzugsweise für C₁-C₄-Alkoxyreste, wie Methoxy; Ethoxy, 1-Propoxy, 2-Propoxy, 1-n-Butoxy, 2-n-Butoxy, 1-i-Butoxy oder 2-i-Butoxy, vorzugsweise Al(OMe)₃, Al(OEt)₃, Al(O-i-Pr)₃, Al(O-s-Bu)₃ insbesondere, Al(O-s-Bu)₃ oder für gleiche oder unterschiedliche, vorzugsweise gleiche, einwertige Alkylreste, wobei mehrere Alkylreste untereinander verbrückt sein können oder ein oder mehrere Alkylreste mit einem oder mehreren der oben genannten Alkoxyresten verbrückt sein können, vorzugsweise für C₁-C₄-Alkylreste, wie Methyl, Ethyl, 1-Propyl, 2-Propyl, 1-n-Butyl, 2-n-Butyl, 1-i-Butyl oder 2-i-Butyl, vorzugsweise Me₃Al, Et₃Al, i-Pr₃Al, Bu₃Al, insbesondere Et₃Al, oder solchen gemischten Alkoxy-Alkyl-Resten.

In einer bevorzugten Ausführungsform kann Verbindung d) ein Alumniumtrialkoxylat, insbesondere Al(O-s-Bu)₃ darstellen.

In einer weiteren bevorzugten Ausführungsform kann Verbindung d) ein Trialyklaluminium, insbesondere Et₃Al darstellen.

Vorteilhaft kann Verbindung d), bezogen auf Ni, in Mengen von 0,01 bis 2, vorzugsweise 0,01. bis 1,5, insbesondere 0,01 bis 1 mol/mol (Gew/Gew) eingesetzt werden.

Die Herstellung von Katalysatorsystemen, enthaltend die Komponenten a), b) und c) ist an sich bekannt; die Herstellung des erfindungsgemäßen Systems kann entsprechend diesen an sich bekannten Verfahren erfolgen.

In Verfahren zur Hydrocyanierung olefinisch ungesättigter Verbindungen in Gegenwart von Ni(0) enthaltenden Katalysatorsystemen können erfindungsgemäß vorteilhaft die vorliegenden Systeme, enthaltend Verbindungen a), b), c) und d) als Ni(0) enthaltenden Katalysatoren eingesetzt werden.

Als olefinisch ungesättigte Verbindung wird im Sinne der vorliegenden Erfindung sowohl eine einzelne olefinisch ungesättigte Verbindung, wie auch ein Gemisch solcher olefinisch ungesättigter Verbindungen verstanden.

Als olefinisch ungesättigte Verbindung kommen Verbindungen in Betracht, die eine oder mehrere, wie zwei, drei oder vier, vorzugsweise eine oder zwei, insbesondere eine Kohlenstoff-Kohlenstoff-Doppelbindung aufweisen. Die olefinisch ungesättigte Verbindung kann vorteilhaft ein verzweigtes oder unverzweigtes Alken, vorzugsweise mit 2 bis 10 Kohlenstoffatomen, oder ein Arylalken, wie ein Monoarylalken oder Bisarylalken, vorzugsweise mit 2 bis 10 Kohlenstoffatomen im Alken-Rückgrat, darstellen.

Solche olefinisch ungesättigte Verbindungen können unsubstituiert sein.

In einer bevorzugten Ausführungsform kann man eine substituierte olefinisch ungesättigte Verbindung einsetzen, vorzugsweise eine olefinisch ungesättigte Verbindung, die eine funktionelle Gruppe, ausgewählt aus der Gruppe bestehend aus -CN, -COOR³¹, -CONR³²R³³
mit R³¹, R³², R³³: unabhängig voneinander, im Falle von R³² und R³³ gleich oder unterschiedlich, H oder Alkyl, vorzugsweise C₁-C₄-Alkyl, wie Methyl, Ethyl, 1-Propyl, 2-Propyl, 1-n-Butyl, 2-n-Butyl, 1-i-Butyl oder 2-i-Butyl
enthält.

In einer weiteren bevorzugten Ausführungsform kann man als substituierte olefinisch ungesättigte Verbindung eine Verbindung der Formel (C₄H₇)-X einsetzen mit X: funktionelle Gruppe, ausgewählt aus der Gruppe bestehend aus -CN, -COOR⁴¹, -CONR⁴²R⁴³
mit R⁴¹, R⁴², R⁴³: unabhängig voneinander, im Falle von R⁴² und R⁴³ gleich oder unterschiedlich, H oder Alkyl, vorzugsweise C₁-C₄-Alkyl, wie Methyl, Ethyl, 1-Propyl, 2-Propyl, 1-n-Butyl, 2-n-Butyl, 1-i-Butyl oder 2-i-Butyl.

In einer weiteren bevorzugten Ausführungsform kann man als olefinisch ungesättigte Verbindung ein verzweigtes, vorzugsweise lineares Pentennitril einsetzen, wie 2-cis-Pentennitril, 2-trans-Pentennitril, 3-cis-Pentennitril, 3-trans-Pentennitril, 4-Pentennitril, E-2-Methyl-2-butennitril, Z-2-Methyl-2-butennitril, 2-Methyl-3-butennitril oder deren Gemische.

In einer besonders bevorzugten Ausführungsform kommt als olefinisch ungesättigte Verbindung 3-Pentennitril, wie 3-cis-Pentennitril oder 3-trans-Pentennitril, 4-Pentennitril oder deren Gemische, in Betracht.

Solche Pentennitrile können nach an sich bekannten Verfahren, beispielsweise durch Hydrocyanierung von Butadien in Gegenwart von Ni(0) enthaltenden Katalysatoren erhalten werden.

Verfahren zur Hydrocyanierung olefinisch ungesättigter Verbindungen in Gegenwart von Ni(0) enthaltenden Katalysatorsystemen sind an sich bekannt. Die erfindungsgemäßen Verfahren können diesen an sich bekannten Verfahren entsprechend durchgeführt werden.

Das bei einer solchen Hydrocyanierung als Produkt erhältliche Adipodinitril ("ADN") oder die durch Hydrierung von ADN erhältlichen Verbindungen 6-Aminocapronitril ("ACN") und Hexamthylendiamin ("HMD") können zur Herstellung von Polyamiden, insbesondere Nylon 6 und Nylon 6.6, eingesetzt werden.

Die Erfindung wird anhand der folgenden, nicht einschränkenden Beispiele näher erläutert.

### Beispiele

Alle Beispiele und Vergleichsbeispiele wurden in einer Argon-Schutzgasatmosphäre durchgeführt.

Nickel(0)-(m-/p-Tolylphosphit)₅₋₇ ("NTP") entspricht einer Lösung aus 2,35 Gew.% Nickel(0) mit 19 Gew.% 3-Pentennitril ("3PN) und 78,65 Gew.% m-/p-Tolylphosphit mit einem m/p-Verhältnis von 2:1.

Als Liganden wurden eingesetzt:

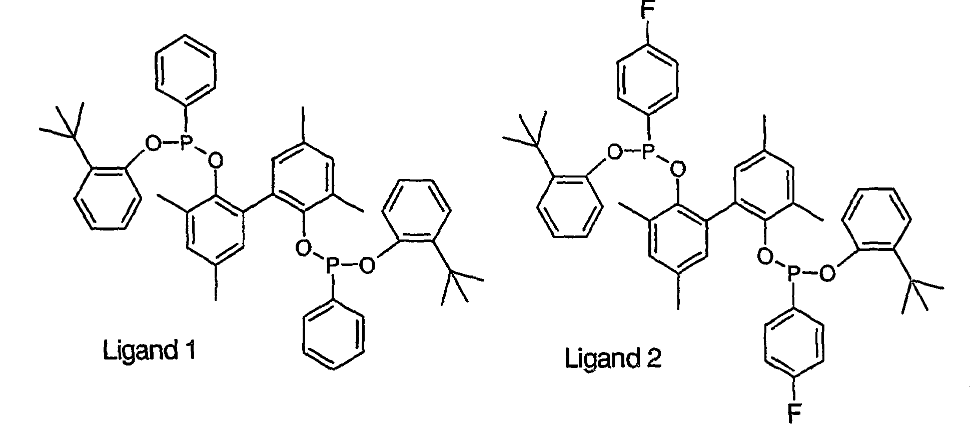

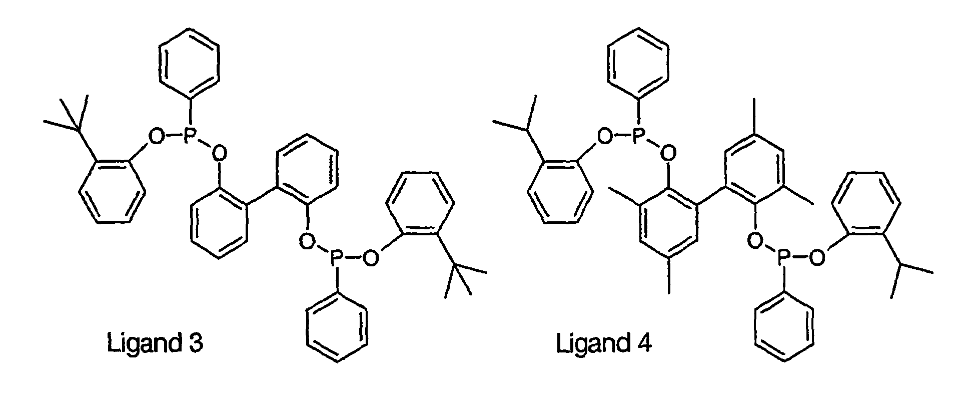

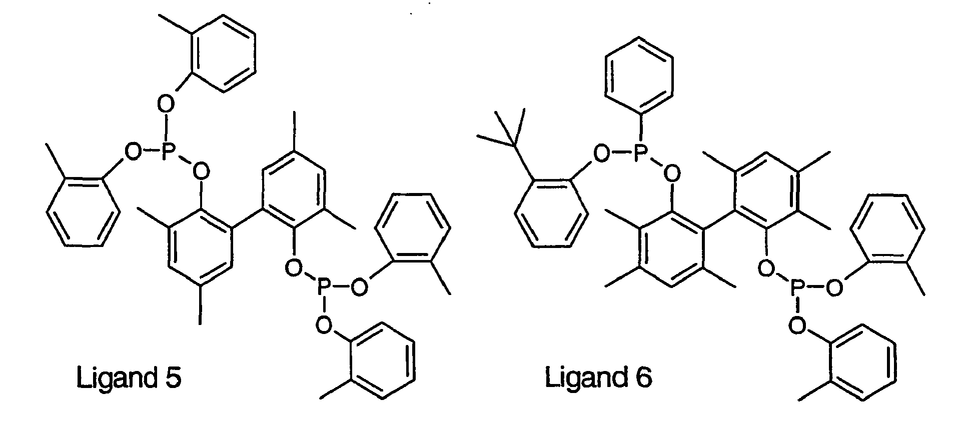

Weiterhin bedeuten "ADN" Adipodinitril, "4PN" 4-Pentennitril und "Ni(COD)₂" Ni(0)-bis-cyclooctadien-Komplex.

### Hydrocyanierung von 3PN zu ADN

### Beispiel 1 (Vergleich), (0,42 mmol Ni(0))

1 Äq. NTP wurde mit 1000 Äq. 3PN und 2 Äq. Ligand 1 versetzt, eine Stunde bei 25°C gerührt und auf 73°C erwärmt. In einem Ar-Trägergasstrom wurden nun 277 Äq. HCN/h*Ni eingegast. Nach 10 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 5,0 | 0,1 | 1,2 | 94,0 |

### Beispiel 2 (Vergleich) (0,42 mmol Ni(0))

1 Äq. NTP wurde mit 1000 Äq. 3PN und 2 Äq. Ligand 1 versetzt, eine Stunde bei 25°C gerührt und auf 73°C erwärmt. Zu dieser Mischung wurde 1 Äq. Et₃Al zugegeben und weitere 5 Min. gerührt. In einem Ar-Trägergasstrom wurden nun 276 Äq. HCN/h*Ni eingegast. Nach 20 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 4,8 | 0,1 | 0,9 | 88,0 |

### Beispiel 3 (Vergleich) (0,42 mmol Ni(0))

1 Äq. NTP wurde mit 1000 Äq. 3PN und 2 Äq. Ligand 1 versetzt, eine Stunde bei 25°C gerührt und auf 60°C erwärmt. Zu dieser Mischung wurde 1 Äq. ZnCl₂ zugegeben und weitere 5 Min. gerührt. In einem Ar-Trägergasstrom wurden nun 351 Äq. HCN/h*Ni eingegast. Nach 65 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 2,1 | 2,0 | 35,8 | 94,8 |

### Beispiel 4 (erfindungsgemäß) (0,47 mmol Ni(0))

1 Äq. NTP wurde mit 1000 Äq. 3PN und 2 Äq. Ligand 1 versetzt, eine Stunde bei 25°C gerührt und auf 60°C erwärmt. Zu dieser Mischung wurde 1 Äq. Et₃Al und 1 Äq. ZnCl₂ zugegeben und weitere 5 Min. gerührt. In einem Ar-Trägergasstrom wurden nun 303 Äq. HCN/h^{*}Ni eingegast. Nach 140 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 0,9 | 3,1 | 64,0 | 95,5 |

### Beispiel 5 (Vergleich): (0,47 mmol Ni(0))

1 Äq.-Ni(COD)₂ wurde mit 3 Äq. Ligand 1 und 1000 Äq. 3PN versetzt, eine Stunde bei 25°C gerührt und auf 73°C erwärmt Zu dieser Mischung wurde 1 Äq. ZnCl₂ zugegeben und weitere 5 Min. gerührt. In einem Ar-Trägergasstrom wurden nun 271 Äq. HCN/h*Ni eingegast. Nach 120 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 1,7 | 3,3 | 50,9 | 94,0 |

### Beispiel 6 (erfindungsgemäß) (0,47 mmol Ni(0))

1 Äq.-Ni(COD)₂ wurde mit 3 Äq. Ligand 1 und 1000 Äq. 3PN versetzt, eine Stunde bei 25°C gerührt und auf 73°C erwärmt. Zu dieser Mischung wurde 1 Äq. Et₃Al und 1 Äq. ZnCl₂ zugegeben und weitere 5 Min. gerührt. In einem Ar-Trägergasstrom wurden nun 268 Äq. HCN/h*Ni eingegast. Nach 150 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 1,4 | 3,4 | 61,3 | 94,7 |

### Beispiel 7 (Vergleich): (0,38 mmol Ni(0))

1 Äq. NTP wurde mit 1000 Äq. 3PN und 2 Äq. Ligand 1 versetzt, eine Stunde bei 25°C gerührt und auf 73°C erwärmt. Zu dieser Mischung wurde 1 Äq. FeCl₂ zugegeben und weitere 5 Min. gerührt. In einem Ar-Trägergasstrom wurden nun 319 Äq. HCN/h*Ni eingegast. Nach 60 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 2,5 | 2,5 | 31,8 | 92,6 |

### Beispiel 8 (erfindungsgemäß) (0,38 mmol Ni(0))

1 Äq. NTP wurde mit 1000 Äq. 3PN und 2 Äq. Ligand 1 versetzt, eine Stunde bei 25°C gerührt und auf 73°C erwärmt. Zu dieser Mischung wurde 0,35 Äq. Et₃Al und 1Äq. FeCl₂ zugegeben und weitere 5 Min. gerührt. In einem Ar-Trägergasstrom wurden nun 324 Äq. HCN/h*Ni eingegast. Nach 110 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | AND | ADN-Selektivität (%) |
|---|---|---|---|
| 1,5 | 3,5 | 50,9 | 93,5 |

### Beispiel 9 (Vergleich) (0,46 mmol Ni(0))

1 Äq.-Ni(COD)₂ wurde mit 3 Äq. Ligand 1 und 1000 Äq. 3PN versetzt, eine Stunde bei 25°C gerührt und auf 73°C erwärmt. Zu dieser Mischung wurde 1 Äq. FeCl₂ zugegeben und weitere 5 Min. gerührt. In einem Ar-Trägergasstrom wurden nun 256 Äq. HCN/h*Ni eingegast. Nach 140 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 1,3 | 3,9 | 61,1 | 94,0 |

### Beispiel 10 (erfindungsgemäß) (0,4 mmol Ni(0))

1 Äq.-Ni(COD)₂ wurde mit 3 Äq. Ligand 1 und 1000 Äq. 3PN versetzt, eine Stunde bei 25°C gerührt und auf 73°C erwärmt. Zu dieser Mischung wurde 0,35 Äq. Et₃Al und 1 Äq. FeCl₂ zugegeben und weitere 5 Min. gerührt. In einem Ar-Trägergasstrom wurden nun 300 Äq. HCN/h*Ni eingegast. Nach 150 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 1,0 | 4,2 | 69,4 | 94,3 |

### Beispiel 11 (Vergleich) (0,43 mmol Ni(0))

1 Äq. NTP wurde mit 1000 Äq. 3PN und 2 Äq. Ligand 1 versetzt, eine Stunde bei 25°C gerührt und auf 73°C erwärmt. Zu dieser Mischung wurde 10 Äq. Al(O-*s*-Bu)₃ und 1 Äq. FeCl₂ zugegeben und weitere 5 Min. gerührt. In einem Ar-Trägergasstrom wurden nun 294 Äq. HCN/h*Ni eingegast. Nach 15 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 1,6 | 0,1 | 0,2 | |

### Beispiel 12 (erfindungsgemäß) (0,42 mmol Ni(0))

1 Äq. NTP wurde mit 1000 Äq. 3PN und 2 Äq. Ligand 1 versetzt, eine Stunde bei 25°C gerührt und auf 73°C erwärmt. Zu dieser Mischung wurde 0,5 Äq. AI(O-s-Bu)₃ und 1Äq. ZnCl₂ zugegeben und weitere 5 Min. gerührt. In einem Ar-Trägergasstrom wurden nun 361 Äq. HCN/h*Ni eingegast. Nach 80 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 1,8 | 3,7 | 51,9 | 93,4 |

### Beispiel 13 (erfindungsgemäß) (0,42 mmol Ni(0))

1 Äq. NTP wurde mit 1000 Äq. 3PN und 2 Äq. Ligand 1 versetzt, eine Stunde bei 25°C gerührt und auf 73°C erwärmt. Zu dieser Mischung wurde 1 Äq. Ti(O-Bu)₄ und 1Äq. ZnCl₂ zugegeben und weitere 5 Min. gerührt. In einem Ar-Trägergasstrom wurden nun 296 Äq. HCN/h^{*}Ni eingegast. Nach 100 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 2,1 | 3,2 | 48,6 | 93,8 |

### Beispiel 14 (Vergleich): (0,3 mmol Ni(0))

1 Äq. NTP wurde mit 300 Äq. 3PN und 2 Äq. Ligand 1 versetzt, eine Stunde bei 25°C gerührt und auf 70°C erwärmt. Zu dieser Mischung wurde 1 Äq. ZnCl₂ zugegeben und weitere 5 Min. gerührt. In einem Ar-Trägergasstrom wurden nun 260 Äq. HCN/h^{*}Ni eingegast. Nach 1, 2, 3, 4, 5 und 10 Minuten wurde eine Probe aus dem Reaktionsgemisch entnommen und gaschromatographisch (GC-Gewichtsprozent, interner Standard: Ethylbenzol) folgende Ergebnisse erhalten:

| Min | 4PN | ADN |
|---|---|---|
| 1 | 2,6 | 3,7 |
| 2 | 3,0 | 7,1 |
| 3 | 3,3 | 9,8 |
| 4 | 3,3 | 12,1 |
| 5 | 3,1 | 15,5 |
| 10 | 2,6 | 27,2 |

### Beispiel 15a (erfindungsgemäß) (0,3 mmol Ni(0))

1 Äq. NTP wurde mit 300 Äq. 3PN und 2 Äq. Ligand 1 versetzt, eine Stunde bei 25°C gerührt und auf 70°C erwärmt. Zu dieser Mischung wurde 1 Äq. Et₃Al und 1 Äq. ZnCl₂ zugegeben und weitere 5 Min. gerührt. In einem Ar-Trägergasstrom wurden nun 260 Äq. HCN/h^{*}Ni eingegast. Nach 1, 2, 3, 4, 5 und 10 Minuten wurde eine Probe aus dem Reaktionsgemisch entnommen und gaschromatographisch (GC-Gewichtsprozent, interner Standard: Ethylbenzol) folgende Ergebnisse erhalten:

| Min | 4PN | ADN |
|---|---|---|
| 1 | 1,9 | 3,6 |
| 2 | 2,2 | 4,5 |
| 3 | 2,4 | 7,7 |
| 4 | 2,6 | 10,9 |
| 5 | 2,6 | 11,5 |
| 10 | 1,5 | 25,4 |

### Beispiel 15b (erfindungsgemäß) (0,3 mmol Ni(0))

1 Äq. NTP wurde mit 300 Äq. 3PN und 2 Äq. Ligand 1 versetzt, eine Stunde bei 25°C gerührt und auf 70°C erwärmt. Zu dieser Mischung wurde 1 Äq. Al(O-s-Bu)₃ und 1 Äq. ZnCl₂ zugegeben und weitere 5 Min. gerührt. In einem Ar-Trägergasstrom wurden nun 265 Äq. HCN/h*Ni eingegast. Nach 1, 2, 3, 4, 5 und 10 Minuten wurde eine Probe aus dem Reaktionsgemisch entnommen und gaschromatographisch (GC-Gewichtsprozent, interner Standard: Ethylbenzol) folgende Ergebnisse erhalten:

| Min | 4PN | ADN |
|---|---|---|
| 1 | 2.7 | 3.2 |
| 2 | 3.1 | 5.2 |
| 3 | 3.3 | 7.8 |
| 4 | 3.4 | 9.6 |
| 5 | 3.3 | 11.9 |
| 10 | 2.5 | 23.9 |

### Vergleichsübersicht

| 4PN-Gehalt [GC-Gewichtsprozent] | | | |
|---|---|---|---|
| | Beispiel 14 | Beispiel 15a | Beispiel 15b |
| Zeit [min.] | Ohne Zusatz | Mit Et₃Al | Mit Al-tri-s-butylat |
| 1 | 2.6 | 1.9 | 2.7 |
| 2 | 3 | 2.2 | 3.1 |
| 3 | 3.3 | 2.4 | 3.3 |
| 4 | 3.3 | 2.6 | 3.4 |
| 5 | 3.1 | 2.6 | 3.3 |
| 10 | 2.6 | 1.5 | 2.5 |

Die erfindungsgemäßen Zusätze zeigen also innerhalb der Messgenauigkeit keine lsomerisierungsaktivität im Sinne von US 4,874,884.

| ADN-Gehalt [GC-Gewichtsprozent] | | | |
|---|---|---|---|
| | Beispiel 14 | Beispiel 15a | Beispiel 15b |
| Zeit [min.] | Ohne Zusatz | Mit Et₃Al | Mit Al-tri-s-butylat |
| 1 | 3.7 | 3.6 | 3.2 |
| 2 | 7.1 | 4.5 | 5.2 |
| 3 | 9.8 | 7.7 | 7.8 |
| 4 | 12.1 | 10.9 | 9.6 |
| 5 | 15.5 | 11.5 | 11.9 |
| 10 | 27.2 | 25.4 | 23.9 |

Die erfindungsgemäßen Zusätze zeigen also innerhalb der Messgenauigkeit keine Beeinflussung der Reaktionsgeschwindigkeit der Hydrocyanierung im Sinne von US 4,874,884.

### Beispiel 16 (Vergleich) (0,29 mmol Ni(0))

1 Äq. NTP wurde mit 1000 Äq. 3PN und 2 Äq. Ligand 2 versetzt, eine Stunde bei 25°C gerührt und auf 60°C erwärmt. Zu dieser Mischung wurde 1 Äq. ZnCl₂ zugegeben und weitere 5 Min. gerührt. In einem Ar-Trägergasstrom wurden nun 314 Äq. HCN/h^{*}Ni eingegast. Nach 50 Min. nimmt der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 1,8 | 1,5 | 25,0 | 94,4 |

### Beispiel 17 (erfindungsgemäß): (0,29 mmol Ni(0))

1 Äq. NTP wurde mit 1000 Äq. 3PN und 2 Äq. Ligand 2 versetzt, eine Stunde bei 25°C gerührt und auf 60°C erwärmt. Zu dieser Mischung wurde 1 Äq. Et₃Al und 1 Äq. ZnCl₂ zugegeben und weitere 5 Min. gerührt. In einem Ar-Trägergasstrom wurden nun 340 Äq. HCN/h*Ni eingegast. Nach 135 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 0,5 | 3,1 | 70,8 | 95,8 |

### Beispiel 18 (Vergleich) (0,43 mmol Ni(0))

1 Äq. NTP wurde mit 1000 Äq. 3PN und 2 Äq. Ligand 3 versetzt, eine Stunde bei 25°C gerührt und auf 60°C erwärmt. Zu dieser Mischung wurde 1 Äq. ZnCl₂ zugegeben und weitere 5 Min. gerührt. In einem Ar-Trägergasstrom wurden nun 297 Äq. HCN/h^{*}Ni eingegast. Nach 65 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 2,2 | 3,2 | 27,0 | 89,4 |

### Beispiel 19 (erfindungsgemäß) (0,43 mmol Ni(0))

1 Äq. NTP wurde mit 1000 Äq. 3PN und 2 Äq. Ligand 3 versetzt, eine Stunde bei 25°C gerührt und auf 60°C erwärmt. Zu dieser Mischung wurde 1 Äq. Et₃Al und 1 Äq. ZnCl₂ zugegeben und weitere 5 Min. gerührt. In einem Ar-Trägergasstrom wurden nun 335 Äq. HCN/h^{*}Ni eingegast. Nach 160 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 0,6 | 8,5 | 70,8 | 89,3 |

### Beispiel 20 (Vergleich): (0,22 mmol Ni(0))

1 Äq. NTP wurde mit 1000 Äq. 3PN und 2 Äq. Ligand 4 versetzt, eine Stunde bei 25°C gerührt und auf 60°C erwärmt. Zu dieser Mischung wurde 1 Äq. ZnCl₂ zugegeben und weitere 5 Min. gerührt. In einem Ar-Trägergasstrom wurden nun 272 Äq. HCN/h^{*}Ni eingegast. Nach 30 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 3,0 | 1,6 | 3,3 | 66,8 |

### Beispiel 21 (erfindungsgemäß) (0,23 mmol Ni(0))

1 Äq. NTP wurde mit 1000 Äq. 3PN und 2 Äq. Ligand 4 versetzt, eine Stunde bei 25°C gerührt und auf 60°C erwärmt. Zu dieser Mischung wurde 1 Äq. Et₃Al und 1Äq. ZnCl₂ zugegeben und weitere 5 Min. gerührt. In einem Ar-Trägergasstrom wurden nun 298 Äq. HCN/h*Ni eingegast. Nach 100 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 2,5 | 4,3 | 41,0 | 90,5 |

### Beispiel 22 (Vergleich) (0,4 mmol Ni(0))

1 Äq. NTP wurde mit 1000 Äq. 3PN und 3 Äq. Ligand 5 versetzt, eine Stunde bei 25°C gerührt und auf 70°C erwärmt. Zu dieser Mischung wurde 1 Äq. ZnCl₂ zugegeben und weitere 5 Min. gerührt. In einem Ar-Trägergasstrom wurden nun 337 Äq. HCN/h^{*}Ni eingegast. Nach 150 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 0,7 | 4,7 | 72,4 | 94,0 |

### Beispiel 23 (erfindungsgemäß) (0,4 mmol Ni(0))

1 Äq. NTP wurde mit 1000 Äq. 3PN und 3 Äq. Ligand 5 versetzt, eine Stunde bei 25°C gerührt und auf 70°C erwärmt. Zu dieser Mischung wurde 1 Äq. Al(O-s-Bu)₃ und 1Äq. ZnCl₂ zugegeben und weitere 5 Min. gerührt. In einem Ar-Trägergasstrom wurden nun 299 Äq. HCN/h^{*}Ni eingegast. Nach 195 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 0 | 4,9 | 90,9 | 94,7 |

### Beispiel 24 (Vergleich): (0,4 mmol Ni(0))

1 Äq. NTP wurde mit 1000 Äq. 3PN und 3 Äq. Ligand 6 versetzt, eine Stunde bei 25°C gerührt und auf 70°C erwärmt. Zu dieser Mischung wurde 1 Äq. ZnCl₂ zugegeben und weitere 5 Min. gerührt. In einem Ar-Trägergasstrom wurden nun 313 Äq. HCN/h*Ni eingegast. Nach 95 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 2,0 | 2,7 | 31,4 | 92,1 |

### Beispiel 25 (erfindungsgemäß) (0,4 mmol Ni(0))

1 Äq. NTP wurde mit 1000 Äq. 3PN und 3 Äq. Ligand 6 versetzt, eine Stunde bei 25°C gerührt und auf 70°C erwärmt. Zu dieser Mischung wurde 1 Äq. Al(O-s-Bu)₃ und 1 Äq. ZnCl₂ zugegeben und weitere 5 Min. gerührt. In einem Ar-Trägergasstrom wurden nun 303 Äq. HCN/h*Ni eingegast. Nach 130 Min. nahm der Ansatz keine HCN mehr auf; es wurde eine Probe aus dem Reaktionsgemisch entnommen und gaschromatographisch (GC-Gewichtsprozent, interner Standard: Ethylbenzol) folgende Ergebnisse erhalten:

| 4PN | MGN | ADN | ADN-Selektivität (%) |
|---|---|---|---|
| 0 | 4,1 | 74,6 | 94,8 |

### Beispiel 26 (vergleich)

Es wurde wie in den Beispielen 14 verfahren mit dem Unterschied, dass am Anfang eine Mischung aus 30 Äqu. 4PN und 270 Äqu. 3PN eingesetzt wurde. Es wurde nach 1, 2, 3, 4, 5 und 10 Minuten eine Probe aus dem Reaktionsgemisch entnommen und gaschromatographisch (GC-Gewichtsprozent, interner Standard: Ethylbenzol) der Gehalt an 4PN ermittelt zur Bestimmung der Beeinflussung der Realctionsgeschwindigkeit der Hydrocyanierung zu ADN der erfindungsgemäßen Zusätze und dabei folgende Ergebnisse erhalten:

| Min | 4PN | ADN |
|---|---|---|
| 1 | 3.4 | 4.1 |
| 2 | 3.4 | 5.7 |
| 3 | 3.3 | 7.4 |
| 4 | 3.4 | 10 |
| 5 | 3.4 | 12.1 |
| 10 | 3 | 24.5 |

### Beispiel 27 (erfindungsgemäß)

Es wurde wie in den Beispielen 15 verfahren mit dem Unterschied, dass am Anfang eine Mischung aus 30 Äqu. 4PN und 270 Äqu. 3PN eingesetzt wurde. Es wurde nach 1, 2, 3, 4, 5 und 10 Minuten eine Probe aus dem Reaktionsgemisch entnommen und gaschromatographisch (GC-Gewichtsprozent, interner Standard: Ethylbenzol) der Gehalt an 4PN ermittelt zur Bestimmung der Beeinflussung der Reaktionsgeschwindigkeit der Hydrocyanierung zu ADN der erfindungsgemäßen Zusätze und dabei folgende Ergebnisse erhalten:

| Min | 4PN | ADN |
|---|---|---|
| 1 | 3.2 | 3.5 |
| 2 | 3.2 | 4.7 |
| 3 | 3.3 | 7.2 |
| 4 | 2.9 | 8.9 |
| 5 | 2.7 | 14.1 |
| 10 | 2.2 | 26.5 |

### Vergleichsübersicht

| 4PN-Gehalt [GC-Gewichtsprozent] | | |
|---|---|---|
| | Beispiel 26 | Beispiel 27 |
| Zeit [min.] | Ohne Zusatz | Mit Et₃Al |
| 1 | 3.4 | 3.2 |
| 2 | 3.4 | 3.2 |
| 3 | 3.3 | 3.3 |
| 4 | 3.4 | 2.9 |
| 5 | 3.4 | 2.7 |
| 10 | 3 | 2.2 |

Die erfindungsgemäßen Zusätze zeigen also innerhalb der Messgenauigkeit keine lsomerisierungsaktivität im Sinne von US 4,874,884.

| ADN-Gehalt [GC-Gewichtsprozent] | | |
|---|---|---|
| | Beispiel 26 | Beispiel 27 |
| Zeit [min.] | Ohne Zusatz | Mit Et₃Al |
| 1 | 4.1 | 3.5 |
| 2 | 5.7 | 4.7 |
| 3 | 7.4 | 7.2 |
| 4 | 10 | 8.9 |
| 5 | 12.1 | 14.1 |
| 10 | 24.5 | 26.5 |

Die erfindungsgemäßen Zusätze zeigen also innerhalb der Messgenauigkeit keine Beeinflussung der Reaktionsgeschwindigkeit der Hydrocyanierung im Sinne von US 4,874,884.

## Patentansprüche

1. Als Katalysator für die Hydrocyanierung von olefinisch ungesättigten Verbindungen geeignetes System, enthaltend
a) Ni(0)
b) Ni(0) als Ligand komplexierende, Phosphite, Phosphonite oder deren Gemische enthaltende Verbindung,
c) eine Lewis-Säure
und
d) eine Verbindung der Formel M Rₙ
wobei c) und d) unterschiedlich sind,
wobei
M: Al oder Ti
R: gleiche oder unterschiedliche einwertige Alkoxyreste, wobei mehrere Alkoxyreste untereinander verbrückt sein können, zusätzlich im Falle von M = Al R gleiche oder unterschiedliche einwertige Alkylreste, wobei mehrere Alkylreste untereinander verbrückt sein können oder ein oder mehrere Alkylreste mit einem oder mehreren der oben genannten Alkoxyresten verbrückt sein können
n: Wertigkeit von M
darstellen.

2. System nach Anspruch 1, wobei R im Falle eines Alkoxyrestes für Methoxy, Ethoxy, 1-Propoxy, 2-Propoxy, 1-n-Butoxy, 2-n-Butoxy, 1-i-Butoxy oder 2-i-Butoxy steht.

3. System nach Anspruch 1, wobei R im Falle eines Alkylrestes für Methyl, Ethyl, 1-Propyl, 2-Propyl, 1-n-Butyl, 2-n-Butyl, 1-i-Butyl oder 2-i-Butyl steht.

4. System nach Anspruch 1 oder 2, wobei Verbindung d) ein Titantetraalkoxylat darstellt.

5. System nach Anspruch 1 oder 2, wobei Verbindung d) ein Aluminiumtrialkoxylat darstellt.

6. System nach Anspruch 1 oder 3, wobei Verbindung d) ein Trialkylaluminium darstellt.

7. System nach den Ansprüchen 1 bis 6, wobei die Reste R in Verbindung d) gleich sind.

8. Verfahren zur Hydrocyanierung einer olefinisch ungesättigten Verbindung in Gegenwart eines Ni(0) enthaltenden Katalysatorsystems, **dadurch gekennzeichnet, dass** man als Ni(0) enthaltenden Katalysatorsystem ein System gemäß den Ansprüchen 1 bis 7 einsetzt.

9. Verfahren nach Anspruch 8, wobei die olefinisch ungesättigte Verbindung eine funktionelle Gruppe, ausgewählt aus der Gruppe bestehend aus -CN, -COOR¹, -CONR²R³
mit R¹, R², R³: unabhängig voneinander, im Falle von R² und R³ gleich oder unterschiedlich, H oder Alkyl,
enthält.

10. Verfahren nach Anspruch 8, wobei man als olefinisch ungesättigte Verbindung eine Verbindung der Formel (C₄H₇)-X
mit X: funktionelle Gruppe, ausgewählt aus der Gruppe bestehend aus -CN, - COOR¹, -CONR²R³
mit R¹, R², R³: unabhängig voneinander, im Falle von R² und R³ gleich oder unterschiedlich, H oder Alkyl,
einsetzt.

11. Verfahren nach Anspruch 8, wobei man als olefinisch ungesättigte Verbindung ein lineares Pentennitril einsetzt.

12. Verfahren nach 8, wobei man als olefinisch ungesättigte Verbindung 3-Pentennitril oder 4-Pentennitril einsetzt.

## Claims

1. A system which is suitable as a catalyst for the hydrocyanation of olefinically unsaturated compounds and comprises
a) Ni(0)
b) a compound which complexes Ni(0) as a ligand and comprises phosphites, phosphonites or mixtures thereof,
c) a Lewis acid
and
d) a compound of the formula M Rₙ
where C) and d) are different,
where
M: Al or Ti
R: identical or different monovalent alkoxy radicals, in which case a plurality of alkoxy radicals may be bonded together, and additionally, in the case that M = Al, R may be identical or different monovalent alkyl radicals, in which case a plurality of alkyl radicals may be bonded together or one or more alkyl radicals may be bonded to one or more of the abovementioned alkoxy radicals,
n: valency of M.

2. The system according to claim 1, wherein R, in the case of an alkoxy radical, is methoxy, ethoxy, 1-propoxy, 2-propoxy, 1-n-butoxy, 2-n-butoxy, 1-isobutoxy or 2-isobutoxy.

3. The system according to claim 1, wherein R, in the case of an alkyl radical, is methyl, ethyl, 1-propyl, 2-propyl, 1-n-butyl, 2-n-butyl, 1-isobutyl or 2-isobutyl.

4. The system according to claim 1 or 2, wherein compound d) is a titanium tetraalkoxide.

5. The system according to claim 1 or 2, wherein compound d) is an aluminum trialkoxide.

6. The system according to claim 1 or 3, wherein compound d) is a trialkylaluminum.

7. The system according to any of claims 1 to 6, wherein the R radicals in compound d) are the same.

8. The process for hydrocyanating an olefinically unsaturated compound in the presence of an Ni(O)-comprising catalyst system, which comprises using a system according to any of claims 1 to 7 as the Ni(O)-comprising catalyst system.

9. The process according to claim 8, wherein the olefinically unsaturated compound comprises a functional group selected from the group consisting of -CN, -COOR¹, -CONR²R³
where R¹, R², R³: each independently, in the case that R² and R³ are the same or different, H or alkyl.

10. The process according to claim 8, wherein the olefinically unsaturated compound used is a compound of the formula (C₄H₇)-X
where X: functional group selected from the group consisting of -CN, -COOR¹, -CONR²R³
where R¹, R², R³: each independently, in the case that R² and R³ are the same or different, H or alkyl.

11. The process according to claim 8, wherein the olefinically unsaturated compound used is a linear pentenenitrile.

12. The process according to claim 8, wherein the olefinically unsaturated compound used is 3-pentenenitrile or 4-pentenenitrile.

## Revendications

1. Système approprié comme catalyseur pour l'hydrocyanation de composés oléfiniquement insaturés, contenant
a) Ni (0),
b) un composé contenant des phosphites, phosphonites ou leurs mélanges, complexant Ni(0) comme ligand,
c) un acide de Lewis,
et
d) un composé de la formule M Rₙ
c) et d) étant différents,
et
M représente Al ou Ti,
R sont des radicaux alcoxy monovalents identiques ou différents, un pont pouvant, en supplément dans le cas où M=Al, être créé entre plusieurs radicaux alcoxy, des radicaux alkyle monovalents identiques ou différents, un pont pouvant être créé entre plusieurs radicaux alkyle, un pont pouvant être créé entre un ou plusieurs radicaux alkyle et un ou plusieurs des radicaux alcoxy cités ci-dessus,
n est la valence de M.

2. Système suivant la revendication 1, dans lequel R représente, dans le cas d'un radical alcoxy, un groupe méthoxy, éthoxy, 1-propoxy, 2-propoxy, 1-n-butoxy, 2-n-butoxy, 1-i-butoxy ou 2-i-butoxy.

3. Système suivant la revendication 1, dans lequel R représente, dans le cas d'un radical alkyle, un groupe méthyle, éthyle, 1-propyle, 2-propyle, 1-n-butyle, 2-n-butyle, 1-i-butyle ou 2-i-butyle.

4. Système suivant la revendication 1 ou 2, dans lequel le composé d) représente un tétraalcoolate de titane.

5. Système suivant la revendication 1 ou 2, dans lequel le composé d) représente un trialcoolate d'aluminium.

6. Système suivant la revendication 1 ou 3, dans lequel le composé d) représente un trialkylaluminium.

7. Système suivant les revendications 1 à 6, dans lequel les radicaux R dans le composé d) sont identiques.

8. Procédé d'hydrocyanation d'un composé oléfiniquement insaturé en présence d'un système catalytique contenant Ni(0), **caractérisé en ce que**, comme système catalytique contenant Ni(0), on met en oeuvre un système suivant les revendications 1 à 7.

9. Procédé suivant la revendication 8, dans lequel le composé oléfiniquement insaturé contient un groupe fonctionnel choisi parmi le groupe constitué de -CN, -COOR¹, -CONR²R³,
où R¹, R², R³ représentent, indépendamment les uns des autres, H ou un groupe alkyle, dans le cas où R² et R³ sont identiques ou différents.

10. Procédé suivant la revendication 8, dans lequel on met en oeuvre, comme composé oléfiniquement insaturé, un composé de la formule (C₄H₇)-X,
où X représente un groupe fonctionnel choisi parmi le groupe constitué de -CN, -COOR¹, -CONR²R³,
où R¹, R², R³ représentent, indépendamment les uns des autres, H ou un groupe alkyle, dans le cas où R² et R³ sont identiques ou différents.

11. Procédé suivant la revendication 8, dans lequel on met en oeuvre, comme composé oléfiniquement insaturé, un pentènenitrile linéaire.

12. Procédé suivant la revendication 8, dans lequel on met en oeuvre, comme composé oléfiniquement insaturé, du 3-pentènenitrile ou du 4-pentènenitrile.
